# EUROPEAN PATENT SPECIFICATION

(11) **EP 3 731 931 B1**
(45) Date of publication and mention of the grant of the patent: **26.11.2025**
(21) Application number: 18913635.1
(22) Date of filing: 21.12.2018
(51) Int. Cl.: A61K 9/20, A61K 9/28, A61K 31/138, A61K 31/222

(54) **MODIFIED RELEASE FORMULATIONS OF FESOTERODINE**
FORMULIERUNGEN VON FESOTERODIN MIT MODIFIZIERTER FREISETZUNG
FORMULATIONS À LIBÉRATION MODIFIÉE DE FÉSOTÉRODINE

(30) Priority: 25.12.2017 TR 201721437
(43) Date of publication of application: 04.11.2020
(73) Proprietor: Sanovel Ilac Sanayi ve Ticaret A.S., Sisli - Istanbul (TR)
(72) Inventor: TÜRKYILMAZ, Ali, 34460 Istanbul (TR); PEHLIVAN AKALIN, Nur, 34460 Istanbul (TR); AKKAYA, Kerim, 34460 Istanbul (TR)
(74) Representative: Genç Ilhan, Oznur
(86) International application number: PCT/TR2018/050855
(87) International publication number: WO 2019/194772

(56) References cited:
- EP-A1- 2 508 173
- EP-B1- 2 029 134
- WO-A1-2015/022703
- US-A1- 2013 236 544
- US-A1- 2013 236 544

## Description

### Field of the Invention

The present invention relates to modified release formulations comprising fesoterodine or a pharmaceutically acceptable salt thereof with at least two sugars or its derivatives as stabilizers.

### Background of the Invention

The chemical name of fesoterodine is (2-[(1R)-3-[bis(1-methylethyl) amino]-1-phenylpropyl]-4-hydroxymethylphenyl isobutyrate or alternatively R-(+)-isobutyric acid 2-(3-diisopropylamino-1-phenylpropyl)-4-hydroxymethylphenyl ester). Its empirical formula is C₃₀H₄₁NO₇, corresponding to a molecular weight of 527.66 having the following structural formula I:

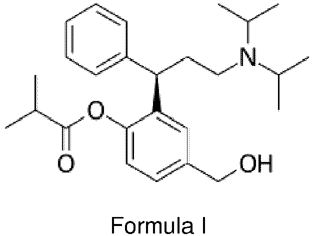

The drug fesoterodine fumarate is the active ingredient in a product being sold as TOVIAZ^{®} tablets to treat urinary incontinence and frequency problems. Inactive ingredients are glyceryl behenate, hypromellose, lactose monohydrate, soya lecithin, microcrystalline cellulose, polyethylene glycol, polyvinyl alcohol, talc, titanium dioxide, and xylitol.

Fesoterodine is known in the art for its potency in treating urinary incontinence. However, fesoterodine may exhibit substantial degradation under stress conditions. It is believed that hydrolyzation and oxidation are among the major mechanisms resulting in degradation.

Synthesis pathways for fesoterodine are described EP 1 077 912 B1. Salts of fesoterodine is described in EP 1 230 209 B1.

WO 2010/043408 discloses a microencapsuled fesoterodine composition which distinct from a homogenous mixture of fesoterodine particle with a matrix, is composed of a particle containing fesoterodine and a shell surrounding fesoterodine containing particle. However, in the case, the stability of compositions is provided a complex-structured pharmaceutical composition.

WO 2015/022703 A1 discloses fesoterodine formulations which comprise a sugar alcohol selected from mannitol, maltitol and lactitol for providing high stability in fesoterodine formulations.

US 2013/236544 A1 discloses a sugar alcohol which is selected from mannitol, maltitol, sucrose, fructose, galactose, lactitol, inositol or erythritol used as a stabilizer in fesoterodine formulations. The weight ratio of fesoterodine to the sugar alcohol is 1:9.

There is thus still a need for a physically and chemically stable composition fesoterodine that are stable against fesoterodine degradation over an extended period of time. In the present invention, stabilizers with binding properties was used, so while the stable formulation is provided without using extra load of excipients, also the formulation provide the desired dissolution profile and excellent pharmacotechnical properties such as flowability, compressibility and homogeneity. Also, the formulation has been developed by using standard techniques which is simple and cost-effective method.

### Detailed Description of the Invention

The main object of the present invention is to provide pharmaceutical formulations of fesoterodine which are more stable against degradation over storage period and also provide the desired modified release of the drug.

Another object of the present invention is to provide a formulation which is characterized by the desired dissolution profile and excellent pharmacotechnical properties, such as flowability, compressibility and homogeneity.

The term "fesoterodine" as used throughout the specification refers to not only fesoterodine, but also its other pharmaceutically acceptable salts, pharmaceutically acceptable solvates, pharmaceutically acceptable hydrates, pharmaceutically acceptable enantiomers, pharmaceutically acceptable derivatives, pharmaceutically acceptable polymorphs or pharmaceutically acceptable prodrugs thereof.

The term "stability" as used herein includes both chemical stability, physical and polymorphic stability.

The term "modified release phase" refers to any pharmaceutical formulation that maintain constant levels of a drug in the patient's bloodstream by releasing the drug over an extended period of time. Modified release is formulated to release the active ingredient gradually and predictably over a 12-hour to 24-hour period. Modified release formulations are also referred to controlled release, sustained release, delayed release, extended release or repeat action systems or mixtures thereof.

In this invention, the modified release formulation comprises fesoterodine and sucrose-fructose as stabilizers wherein the ratio of fesoterodine to sucrose-fructose mixture is in the range of between 1:15 and 15:1 by weight.

In one embodiment of this present invention, the amount of fesoterodine is between 0.5% and 25.0% w/w of the composition, preferably it is between 0.5% and 15.0% w/w of the composition.

In one embodiment of this present invention, the ratio of fesoterodine to stabilizers is between 1:15 and 15:1 by weight.

Fructose is more soluble than other sugars and hard to crystallize because it is more hygroscopic and holds onto water stronger than the others. This means that fructose can be used to extend the shelf life of composition more than other sugars. But, fructose has some side-effects when is not used in reasonable amounts. Thereof, fructose-sucrose mixture is used. Surprisingly, it has been found that using both fructose and sucrose as the stabilizer provides improved stability and better dissolution rate to the formulation and prevented degradation of fesoterodine.

In the present invention, the modified release formulation comprises the fructose-sucrose mixture as a stabilizer.

According to one embodiment in the present invention, the fructose-sucrose mixture is a stabilizer which have binder properties. In addition, it further enhances excellent pharmacotechnical properties (flowability, compressibility and homogeneity).

In one embodiment of this present invention, the amount of the fructose-sucrose mixture is between 3.0% and 35.0% by weight of the composition, preferably it is between 10.0% and 31.0% by weight., more preferably it is between 14.0% and 29.0% by weight.

In one embodiment of this present invention, the ratio of fructose to sucrose is in the range of between 1:50 and 50:1 by weight, preferably between 40:1 and 1:40 by weight, more preferably between 35:1 and 1:35 by weight. These ratios are important in order to provide stability in the present formulation.

The pharmaceutically acceptable excipient which is used in the formulation and process for preparation of the formulation of the present invention must be compatible with fesoterodine.

According to another embodiment of this invention, modified release formulation further comprises at least one pharmaceutically acceptable excipient which is selected from the group comprising diluents, rate controlling polymers, lubricants, coating agents or the mixtures thereof.

Suitable diluents are selected from the group lactose monohydrate, microcrystalline cellulose, calcium carbonate, calcium phosphate dibasic, calcium phosphate tribasic, calcium sulfate, microcrystalline silicified cellulose, powdered cellulose, dextrates, dextrose, fructose, lactitol. lactose anhydrous, lactose dihydrate or mixtures thereof. The amounts of diluents are between 10.0% and 28.0% w/w of the composition, preferably the amounts of diluents are between 13.0% and 25.0% w/w of the composition.

Modified release formulations are preferred. At this invention, formulation is prepared using rate controlling polymers.

Suitable rate controlling polymers are selected from the group comprising hydroxypropylmethyl cellulose, carbomer, xanthan gum, polyethylene oxides, glyceryl dibehenate, hydroxyethyl cellulose, hydroxypropyl cellulose, hydroxyethyl methyl cellulose, methylcellulose, carboxymethylcellulose and its salts, polyacrylates, , methylacrylates, polyethylene glycols, starch derivatives, galactomannans, polysaccharides, polyalcohols, acrylic acid or its derivatives, glycerol monostearate, polyanhydrides, methylacrylates, polyamides, polycarbonates, polyalkylene, polyalkylene glycols, polyalkylene oxides, polyvinyl alcohols, polyvinyl ethers, polyvinylpyrrolidone, polymers of acrylic and methacrylic esters, polylactides, polyorthoesters, poly(fumaric acid) or mixtures thereof.

Particularly, the preferred rate controlling polymers are selected from a group comprising of hydroxypropylmethyl cellulose, carbomer, xanthan gum, polyethylene oxides, glyceryl dibehenate or mixture thereof. The selected controlled release polymers are preferably present in an amount that allows for the formation of a gel matrix from which the active ingredient is gradually released.

The amount of the rate controlling polymers are in the modified release formulation between 10.0% and 60.0% w/w of the formulation, preferably between 20.0% and 55.0% w/w of the formulation

In one embodiment of this present invention, the rate controlling polymers are hydroxypropylmethyl cellulose, polyethylene oxides and glyceryl dibehenate.

In one embodiment of this present invention, the ratio of fesoterodine to hydroxypropylmethyl cellulose is in the range of between 10:1 and 1:10 by weight, preferably this ratio is in the range of between 8:1 and 1:8 by weight. While this ratio helps to protect improved stability in the present formulation, at the same time provide the desired modified release of the drug.

The amount of the hydroxypropylmethyl cellulose is in the pharmaceutical composition between 10.0% and 40.0% w/w of the formulation.

Suitable lubricants are selected from the group from talc, calcium silicate, powdered cellulose, starch, colloidal silicon dioxide or mixtures thereof. Lubricants are between 0.01% and 4.0% w/w of the formulation.

In another embodiment of this present invention, the compositions comprise a film coating. A film coat on the tablet protects from moisture and further contributes to the ease with which it can be swallowed. Preferably, a moisture barrier film coating is used in order to minimize the degradation of fesoterodine due to moisture.

Suitable coating agents are selected from the group comprising polyvinyl alcohol (PVA), talc, polymethacrylates, hydroxypropyl methylcellulose, sodium lauryl sulfate, glyceryl monocaprylocaprate, lactose monohydrate, hydroxypropyl cellulose, polyethylene glycol (PEG), polyvinyl alcohol-polyethylene glycol copolymers, ethylcellulose dispersions, polyvinylpyrrolidone, polyvinylpyrrolidone-vinyl acetate copolymer (PVP-VA), all kinds of Opadry^{®}, pigments, dyes, titanium dioxide, macrogol, coloring agent or mixtures thereof.

Suitable coloring agents are selected from the group comprising ferric oxide, titanium dioxide, Food, Drug & Cosmetic (FD&C) dyes (such as; FD&C blue, FD&C green, FD&C red, FD&C yellow, FD&C lakes), poncau, indigo Drug & Cosmetic (D&C) blue, indigotine FD&C blue, carmoisine indigotine (indigo Carmine); iron oxides (such as; iron oxide red, yellow, black), quinoline yellow, flaming red, carmine, carmoisine, sunset yellow or mixtures thereof.

Solid oral dosage is used for having effective stability and bioavailability. In another embodiment of the present invention, the modified release formulation is in a solid oral dosage form.

The modified release formulation according to the present invention is in the form of tablet, capsule, pastilles, strip.

The modified release formulation according to the present invention is in the form of tablet.

Tablet is film-coated tablets, bilayer tablets, inlay tablets, orally disintegrating tablets, compressed tablets, coated or uncoated tablets, multilayer tablets, mini tablets, buccal tablets, sublingual tablets, effervescent tablets, immediate release tablets, modified release tablets, gastric disintegrating tablets, chewable tablets, dispersing tablets or lozenges.

Preferably, the modified release formulation is in the form of film-coated tablet.

In this present invention, the tablet comprises at least one type of particle, for example; mini-tablet, pellets, agglomerates, granules, powder, liposomes, sphericles or mixtures thereof.

An embodiment of this present invention, each type of particle comprises at least one active agent.

In one embodiment of the invention, the modified release formulation comprises;
0.5% - 25.0% by weight of fesoterodine
3.0% - 35.0% by weight of fructose
0.05% - 20.0% by weight of sucrose
5.0% - 32.0% by weight of lactose monohydrate
1.0% - 25.0% by weight of glyceryl dibehenate
5.0% - 35.0% by weight of microcrystalline cellulose
0.05% - 10.0% by weight of talc
10.0% - 40.0% by weight of hydroxypropyl methylcellulose
1.0% - 25.0% by weight of polyethylene oxides
2.0% - 10.0% by weight of coating

In one embodiment of the invention, the modified release formulation comprises;
0.5% - 5.0% by weight of fesoterodine
19.0% - 25.0% by weight of fructose
0.1% - 3.0% by weight of sucrose
15.0% - 21.0% by weight of lactose monohydrate
3.0% - 10.0% by weight of glyceryl dibehenate
12.0% - 20.0% by weight of microcrystalline cellulose
0.1% - 3.0% by weight of talc
10.0% - 40.0% by weight of hydroxypropyl methylcellulose
4.0% - 12.0% by weight of polyethylene oxides
3.0% - 8.0% by weight of coating

The modified release formulation of the present invention may be prepared by direct compression, wet or dry granulation, hot melt granulation, hot melt extrusion, fluidized bed granulation, extrusion/spheronization, slugging, spray drying or solvent evaporation.

Preferably, the modified release formulation is prepared wet granulation which is simple and cost-effective method. Also, this process helps to provide stability and dissolution profile of the tablet.

Process for preparing the modified release formulation comprises the following steps;
a) Mixing fesoterodine, fructose and sucrose
b) Granulating the mixture with water then, sieving
c) Drying the mixture at 40°C until the humidity is less than 0.5%, then sieving the mixture
d) Adding lactose monohydrate, glyceryl dibehenate, microcrystalline cellulose, hydroxypropyl methylcellulose, polyethylene oxides and then mixing
e) Then, adding talc and mixing
f) Then, pressing to form tablet
g) Coating tablets with coating agent

It has been found that using wet granulation process, wherein granulating fesoterodine and the fructose-sucrose mixture as a stabilizer with the addition of excipients for wet granulation showed an improved stability of fesoterodine in the granulate and in the pharmaceutical formulation.

Also, it has been found that when the formulation is prepared with using water based granulation, resulting formulations possess improved stability.

The given below examples describes the modified release formulation comprising fesoterodine.

### Example 1: Modified release formulation comprising fesoterodine (not part of the invention)

| **agents** | **Amount (% by weight of the total)** |
|---|---|
| Fesoterodine | 0.5% to 8.0% |
| Rate controlling polymers | 10.0% to 60.0% |
| Diluent | 18.0% to 35.0% |
| Stabilizers | 15.0% to 28.0% |
| Lubricant | 0.3% to 1.2% |
| Coating agent | 3.0% to 10.0% |
| **Total** | **100** |

### Process for example 1:

The process for preparation of the modified release formulation comprises the following steps:
a) Mixing fesoterodine and at least two stabilizers
b) Granulating the mixture with water then, sieving
c) drying the mixture at 40°C until the humidity is less than 0.5%, then sieving the mixture
d) Adding diluents, rate controlling polymers and then mixing
e) Then, adding lubricants and mixing
f) Then, pressing to form tablet
g) Optionally, coating tablets with coating agent

### Example 2: Modified release formulation comprising fesoterodine

| **agents** | **Amount (% by weight of the total)** |
|---|---|
| Fesoterodine | 0.5% to 15.0% |
| Fructose | 6.0% to 30.0% |
| Sucrose | 0.1% to 15.0% |
| Lactose monohydrate | 10.0% to 28.0% |
| Glyceryl dibehenate | 1.0% to 15.0% |
| Microcrystalline cellulose | 7.0% to 25.0% |
| Talc | 0.1% to 10.0% |
| Hydroxypropyl methylcellulose | 10.0% to 40.0% |
| Polyethylene oxides | 1.0% to 15.0% |
| Coating | 3.0% to 10.0% |
| **Total** | **100** |

### Process for example 2:

The process for preparation of the modified release formulation comprises the following steps:
a) Mixing fesoterodine, fructose and sucrose
b) Granulating the mixture with water then, sieving
c) Drying the mixture at 40°C until the humidity is less than 0.5%, then sieving the mixture
d) Adding lactose monohydrate, glyceryl dibehenate, microcrystalline cellulose, hydroxypropyl methylcellulose, polyethylene oxides and then mixing
e) Then, adding talc and mixing
f) Then, pressing to form tablet
g) Optionally, coating tablets with coating agent

### Example 3: Modified release formulation comprising fesoterodine

| **agents** | **Amount (% by weight of the total)** |
|---|---|
| Fesoterodine | 2.5% |
| Fructose | 21.8% |
| Sucrose | 0.6% |
| Lactose monohydrate | 18.0% |
| Glyceryl dibehenate | 6.25% |
| Microcrystalline cellulose | 16.0% |
| Talc | 0.7% |
| Hydroxypropyl methylcellulose | 18.0% |
| Polyethylene oxides | 7.8% |
| Coating | 6.25% |
| **Total** | **100** |

### Process for example 3:

The process for preparation of the modified release formulation comprises the following steps:
h) Mixing fesoterodine, fructose and sucrose
i) Granulating the mixture with water then, sieving
j) drying the mixture at 40°C until the humidity is less than 0.5%, then sieving the mixture
k) Adding lactose monohydrate, glyceryl dibehenate, microcrystalline cellulose, hydroxypropyl methylcellulose, polyethylene oxides and then mixing
l) Then, adding talc and mixing
m) Then, pressing to form tablet
n) Optionally, coating tablets with coating agent

## Claims

1. A modified release formulation comprising fesoterodine and sucrose-fructose mixture as stabilizers wherein the ratio of fesoterodine to sucrose-fructose mixture is in the range of between 1:15 and 15:1 by weight.

2. The modified release formulation according to claim 1, wherein the amount of the fructose-sucrose mixture is between 3.0% and 35.0% w/w of the total composition.

3. The modified release formulation according to claim 1, wherein the ratio of fructose to sucrose is in the range of between 1:50 and 50:1 by weight, preferably between 40:1 and 1:40 by weight.

4. The modified release formulation according to claim 1, further comprising at least one pharmaceutically acceptable excipient which is selected from the group comprising diluents, rate controlling polymers, lubricants, coating agents or mixtures thereof.

5. The modified release formulation according to claim 4, wherein rate controlling polymers are selected from the group comprising hydroxypropylmethyl cellulose, carbomer, xanthan gum, polyethylene oxides, glyceryl dibehenate, hydroxyethyl cellulose, hydroxypropyl cellulose, hydroxyethyl methyl cellulose, methylcellulose, carboxymethylcellulose and its salts, polyacrylates, , methylacrylates, polyethylene glycols, starch derivatives, galactomannans, polysaccharides, polyalcohols, acrylic acid or its derivatives, glycerol monostearate, polyanhydrides, methylacrylates, polyamides, polycarbonates, polyalkylene, polyalkylene glycols, polyalkylene oxides, polyvinyl alcohols, polyvinyl ethers, polyvinylpyrrolidone, polymers of acrylic and methacrylic esters, polylactides, polyorthoesters, poly(fumaric acid) or mixtures thereof.

6. The modified release formulation according to claim 5, wherein the rate controlling polymers are selected from a group comprising of hydroxypropylmethyl cellulose, carbomer, xanthan gum, polyethylene oxides, glyceryl dibehenate or mixture thereof.

7. The modified release formulation according to claim 6, wherein the amount of the rate controlling polymers in the pharmaceutical composition is between 10.0% and 60.0% w/w, preferably between 20.0% and 55.0% w/w of the composition.

8. The modified release formulation according to claim 6, wherein rate controlling polymers are hydroxypropylmethyl cellulose, polyethylene oxides or glyceryl dibehenate or their mixtures.

9. The modified release formulation according to claim 8, wherein the ratio of fesoterodine to hydroxypropylmethyl cellulose is in the range of between 10:1 and 1:10 by weight by weight.

10. The modified release formulation according to claim 8 or 9, wherein the amount of the hydroxypropylmethyl cellulose in the pharmaceutical composition is between 10.0% and 40.0% w/w of the composition.

11. The modified release formulation according to any preceding claims, the formulation comprising;
0.5% - 25.0% by weight of fesoterodine
3.0% - 35.0% by weight of fructose
0.05% - 20.0% by weight of sucrose
5.0% - 32.0% by weight of lactose monohydrate
1.0% - 25.0% by weight of glyceryl dibehenate
5.0% - 35.0% by weight of microcrystalline cellulose
0.05% - 10.0% by weight of talc
10.0% - 40.0% by weight of hydroxypropyl methylcellulose
1.0% - 25.0% by weight of polyethylene oxides
2.0% - 10.0% by weight of coating

12. Process for preparing the modified release formulation according to claim 11, the formulation comprising the following steps;
a) Mixing fesoterodine, fructose and sucrose
b) Granulating the mixture with water then, sieving
c) Drying the mixture, then sieving the mixture
d) Adding lactose monohydrate, glyceryl dibehenate, microcrystalline cellulose, hydroxypropyl methylcellulose, polyethylene oxides and then mixing
e) Then, adding talc and mixing
f) Then, pressing to form tablet
g) Coating tablets with coating agent

13. The modified release formulation according to any preceding claims, wherein the formulation is in the form of tablet, capsule, pastilles, strip.

## Patentansprüche

1. Modifizierte Freisetzungsformulierung, umfassend Fesoterodin und eine Saccharose-Fructose-Mischung als Stabilisatoren, wobei das Verhältnis von Fesoterodin zu Saccharose-Fructose-Mischung im Bereich zwischen 1:15 und 15:1, bezogen auf das Gewicht, liegt.

2. Modifizierte Freisetzungsformulierung nach Anspruch 1, wobei die Menge der Fructose-Saccharose-Mischung zwischen 3,0 % und 35,0 % Gew.-% der Gesamtzusammensetzung beträgt.

3. Modifizierte Freisetzungsformulierung nach Anspruch 1, wobei das Verhältnis von Fructose zu Saccharose im Bereich von 1:50 bis 50:1, gewichtsbezogen, vorzugsweise zwischen 40:1 und 1:40, gewichtsbezogen, liegt.

4. Modifizierte Freisetzungsformulierung nach Anspruch 1, die ferner mindestens einen pharmazeutisch akzeptablen Hilfsstoff umfasst, der aus der Gruppe ausgewählt ist, die Verdünnungsmittel, geschwindigkeitssteuernde Polymere, Gleitmittel, Beschichtungsmittel oder Mischungen davon umfasst.

5. Modifizierte Freisetzungsformulierung nach Anspruch 4, wobei die geschwindigkeitssteuernden Polymere ausgewählt sind aus der Gruppe bestehend aus Hydroxypropylmethylcellulose, Carbomer, Xanthangummi, Polyethylenoxiden, Glyceryldibehenat, Hydroxyethylcellulose, Hydroxypropylcellulose, Hydroxyethylmethylcellulose, Methylcellulose, Carboxymethylcellulose und deren Salze, Polyacrylate, Methylacrylate, Polyethylenglykole, Stärkederivate, Galactomannane, Polysaccharide, Polyalkohole, Acrylsäure oder deren Derivate, Glycerinmonostearat, Polyanhydride, Methylacrylate, Polyamide, Polycarbonate, Polyalkylen, Polyalkylenglykole, Polyalkylenoxide, Polyvinylalkohole, Polyvinyläther, Polyvinylpyrrolidon, Polymere von Acryl- und Methacrylestern, Polylactide, Polyorthoester, Poly(fumarsäure) oder Mischungen davon.

6. Modifizierte Freisetzungsformulierung nach Anspruch 5, wobei die geschwindigkeitssteuernden Polymere aus einer Gruppe ausgewählt sind, die Hydroxypropylmethylcellulose, Carbomer, Xanthangummi, Polyethylenoxide, Glyceryldibehenat oder Mischungen davon umfasst.

7. Modifizierte Freisetzungsformulierung nach Anspruch 6, wobei die Menge der geschwindigkeitssteuernden Polymere in der pharmazeutischen Zusammensetzung zwischen 10,0 % und 60,0 % Gew./Gew., vorzugsweise zwischen 20,0 % und 55,0 % Gew./Gew. der Zusammensetzung beträgt.

8. Modifizierte Freisetzungsformulierung nach Anspruch 6, wobei die geschwindigkeitssteuernden Polymere Hydroxypropylmethylcellulose, Polyethylenoxide oder Glyceryl-Dibehenat oder Mischungen davon sind.

9. Modifizierte Freisetzungsformulierung nach Anspruch 8, wobei das Verhältnis von Fesoterodin zu Hydroxypropylmethylcellulose im Bereich zwischen 10:1 und 1:10, bezogen auf das Gewicht, liegt.

10. Modifizierte Freisetzungsformulierung gemäß Anspruch 8 oder 9, wobei die Menge an Hydroxypropylmethylcellulose in der pharmazeutischen Zusammensetzung zwischen 10,0 % und 40,0 % Gew./Gew. der Zusammensetzung beträgt.

11. Modifizierte Freisetzungsformulierung gemäß einem der vorstehenden Ansprüche, wobei die Formulierung umfasst: 0,5 % bis 25,0 Gew.-% Fesoterodin 3,0 % bis 35,0 Gew.-% Fructose 0,05 % bis 20,0 Gew.-% Saccharose 5,0 % - 32,0 Gew.-% LactoseMonohydrat 1,0 % - 25,0 Gew.-% Glyceryl-Dibehenat 5,0 % - 35,0 Gew.-% mikrokristalline Cellulose 0,05 - 10,0 Gew.-% Talk 10,0 - 40,0 Gew.-% Hydroxypropylmethylcellulose 1,0 - 25,0 Gew.-% Polyethylenoxide 2,0 - 10,0 Gew.-% Überzug.

12. Verfahren zur Herstellung der Formulierung mit modifizierter Freisetzung gemäß Anspruch 11, wobei die Formulierung die folgenden Schritte umfasst:
a) Mischen von Fesoterodin, Fructose und Saccharose
b) Granulieren der Mischung mit Wasser und anschließendes Sieben
c) Trocknen der Mischung, dann Sieben der Mischung
d) Zusetzen von Lactosemonohydrat, Glyceryl-Dibehenat, mikrokristalliner Cellulose, Hydroxypropylmethylcellulose, Polyethylenoxiden und anschließendes Mischen
e) Anschließend Talkum hinzufügen und mischen
f) Anschließend zu Tabletten pressen
g) Tabletten mit einem Überzugsmittel überziehen

13. Die modifizierte Freisetzungsformulierung gemäß einem der vorstehenden Ansprüche, wobei die Formulierung in Form von Tabletten, Kapseln, Pastillen oder Streifen vorliegt.

## Revendications

1. Formulation à libération modifiée comprenant de la fésotérodine et un mélange de saccharose et de fructose comme stabilisants, dans laquelle le rapport entre la fésotérodine et le mélange de saccharose et de fructose est compris entre 1:15 et 15:1 en poids.

2. Formulation à libération modifiée selon la revendication 1, dans laquelle la quantité du mélange fructose-saccharose est comprise entre 3,0 % et 35,0 % en poids de la composition totale.

3. Formulation à libération modifiée selon la revendication 1, dans laquelle le rapport entre le fructose et le saccharose est compris entre 1:50 et 50:1 en poids, de préférence entre 40:1 et 1:40 en poids.

4. Formulation à libération modifiée selon la revendication 1, comprenant en outre au moins un excipient pharmaceutiquement acceptable choisi dans le groupe comprenant les diluants, les polymères contrôlant la vitesse, les lubrifiants, les agents d'enrobage ou leurs mélanges.

5. Formulation à libération modifiée selon la revendication 4, dans laquelle les polymères régulateurs de vitesse sont choisis dans le groupe comprenant l'hydroxypropylméthylcellulose, le carbomère, la gomme xanthane, les polyéthylène oxydes, le dibehenate de glycéryle, l'hydroxyéthylcellulose, l'hydroxypropylcellulose, l'hydroxyéthylméthylcellulose, la méthylcellulose, la carboxyméthylcellulose et ses sels, les polyacrylates, les méthylacrylates, les polyéthylèneglycols, les dérivés d'amidon, les galactomannanes, les polysaccharides, les polyalcools, l'acide acrylique ou ses dérivés, le monostéarate de glycérol, les polyanhydrides, méthylacrylates, polyamides, polycarbonates, polyalkylènes, polyalkylèneglycols, polyalkylèneoxydes, alcools polyvinyliques, éthers polyvinyliques, polyvinylpyrrolidone, polymères d'esters acryliques et méthacryliques, polylactides, polyorthoesters, poly(acide fumarique) ou leurs mélanges.

6. Formulation à libération modifiée selon la revendication 5, dans laquelle les polymères contrôlant la vitesse sont choisis parmi un groupe comprenant l'hydroxypropylméthylcellulose, le carbomère, la gomme xanthane, les polyéthylène oxydes, le dibehenate de glycéryle ou un mélange de ceux-ci.

7. Formulation à libération modifiée selon la revendication 6, dans laquelle la quantité de polymères contrôlant la vitesse dans la composition pharmaceutique est comprise entre 10,0 % et 60,0 % p/p, de préférence entre 20,0 % et 55,0 % p/p de la composition.

8. Formulation à libération modifiée selon la revendication 6, dans laquelle les polymères contrôlant la vitesse sont l'hydroxypropylméthylcellulose, les polyéthylène oxydes ou le dibehénate de glycéryle ou leurs mélanges.

9. Formulation à libération modifiée selon la revendication 8, dans laquelle le rapport entre la fésotérodine et l'hydroxypropylméthylcellulose est compris entre 10:1 et 1:10 en poids.

10. Formulation à libération modifiée selon la revendication 8 ou 9, dans laquelle la quantité d'hydroxypropylméthylcellulose dans la composition pharmaceutique est comprise entre 10,0 % et 40,0 % en poids de la composition.

11. Formulation à libération modifiée selon l'une quelconque des revendications précédentes, la formulation comprenant : 0,5 % à 25,0 % en poids de fésotérodine 3,0 % à 35,0 % en poids de fructose 0,05 % à 20,0 % en poids de saccharose 5,0 % à 32,0 % en poids de lactose monohydraté 1,0 % à 25,0 % en poids de dibehenate de glycéryle 5,0 % - 35,0 % en poids de cellulose microcristalline 0,05 % - 10,0 % en poids de talc 10,0 % - 40,0 % en poids d'hydroxypropylméthylcellulose 1,0 % - 25,0 % en poids de polyéthylène oxydes 2,0 % - 10,0 % en poids d'enrobage.

12. Procédé de préparation de la formulation à libération modifiée selon la revendication 11, la formulation comprenant les étapes suivantes :
a) Mélange de fésotérodine, de fructose et de saccharose
b) Granuler le mélange avec de l'eau, puis tamiser
c) Sécher le mélange, puis tamiser le mélange
d) Ajouter du lactose monohydraté, du dibehenate de glycéryle, de la cellulose microcristalline, de l'hydroxypropylméthylcellulose, des oxydes de polyéthylène, puis mélanger
e) Ensuite, ajouter le talc et mélanger
f) Ensuite, presser pour former le comprimé
g) Enrober les comprimés d'un agent d'enrobage

13. La formulation à libération modifiée selon l'une quelconque des revendications précédentes, dans laquelle la formulation se présente sous la forme d'un comprimé, d'une capsule, de pastilles ou d'une bande.
